# EUROPEAN PATENT APPLICATION

(11) **EP 2 687 605 A1**
(43) Date of publication of application: **22.01.2014**
(21) Application number: 12305883.6
(22) Date of filing: 19.07.2012
(51) Int. Cl.: C12N 15/82, A01H 5/00, A01H 4/00

(54) **Method for performing homologous recombination**

(71) Applicant: Biogemma, 75001 Paris (FR)
(72) Inventor: Paul, Wyatt, 63720 Chappes (FR); Ayar, Ayhan, 63000 Cermont-Ferrand (FR); White, Charles, 63170 Pérignat-Les-Sarliève (FR); Gallego, Maria-Eugenia, 63170 Pérignat-Les-Sarliève (FR)
(74) Representative: Flesselles, Bruno F.G.

(57) **Abstract**

The invention relates to a method for obtaining a plant in which a homologous recombination event has occurred, preferably resulting in targeting gene insertion in the genome of said plant, by regeneration of the plant from *in vitro* culture.

## Description

The invention relates to the field of transgenic plants, and in particular to the field of gene targeting in plants.

Transgenesis offers the possibility to insert a known DNA sequence into the genome of an organism to introduce new heritable characters. It is commonly used in research to investigate gene function and by plant biotechnology companies to improve agronomic traits.

However random insertion of the transgene in the genome can result in mutations caused by the insertion of the transgene in an endogenous gene (Krysan et al., 1999), potential production of unintended peptides or variable expression of the transgene due to the genomic environment of the transgene (Matzke and Matzke, 1998).

Thus there are currently considerable efforts undertaken by plant biotechnology companies to develop efficient technologies to develop gene targeting (GT) to produce GM crops with transgenes located at defined and predetermined positions in the plant genome. Exploiting the cellular Homologous Recombination (HR) machinery, GT allows the exchange of genetic information between homologous DNA sequences and can be used to precisely modify the genome. The integration of transgenes flanked by sequences homologous to the desired genomic insertion site permits efficient and routine gene targeting in prokaryotes and fungi but in higher plants GT is very inefficient with around 1 targeted insertion in 10000 (Hanin et al., 2001; Paszkowski et al., 1988).

HR and Non Homologous End Joining (NHEJ) are triggered to repair DNA Double Strand Breaks (DSBs). These lesions are formed accidentally by genotoxic stresses (Hanin and Paszkowski, 2003; Khanna et al., 2001), or in a programmed manner - for example during meiosis by the Spo11 complex (Grelon et al., 2001). NHEJ repair links the two ends of the DSB and is frequently accompanied by the creation of mutations at the site of the repair. HR copies an endogenous (different allele or stably inserted transgene) or exogenous sequence template (transiently inserted transgene) with homology on either side of the break and allows a precise modification of the genome (Puchta et al., 1996).

Usually the transgene is integrated into a DSB (Tzfira et al., 2004) which occurred randomly in the plant genome and thus no shares no homologous sequence with the transgene. Intregration is thus by NHEJ. The fact that the DSB initiates HR and that a DSB at a specific genomic location presenting homologous sequence to transgene increases significantly the recombination rate at this site (Puchta et al., 1993; Szostak et al., 1983), has led to development of tools such as meganucleases, Zinc-Finger Nucleases (Shukla et al., 2009) and Transcription Activator-Like Effector Nucleases (Christian et al., 2010) for gene targeting.

These endonucleases used to create a DSB at the Target Locus (TL), have been developed in order to modify the TL by mutation using NHEJ (Yang et al., 2009) or by precise sequence modification using HR (Tzfira and White, 2005). For example, the mitochondrial I-Scel meganuclease from *Saccharomyces cerevisae* (Jacquier and Dujon, 1985) has been successfully used in plants to perform GT (D'Halluin et al., 2008; Puchta et al., 1996). For example, in tobacco, the cleavage of the chromosomal artificial TL containing an I-Scel restriction site by I-Scel increases recombination between the TL and the transforming T-DNA around 100-fold (Puchta et al., 1996). The enzyme required to produce the DSB can be introduced into the organism / cell via stable or transient transformation. For example, I-Scel has been introduced into plants via Agrobacterium-mediated retransformation of a TL line or by crossing lines stably expressing I-*Sce*l to a TL. In the latter case, the use of an inducible I-*Sce*I can allow the creation of the DSB at the specified moment. For example, in *Arabidopsis*, application of the glutocorticoid, dexamethasone, induced the activity of an I-*Sce*I protein fused to the rat Glucocorticoid Receptor (GR) domain. (Wehrkamp-Richter et al., 2009). The GR domain sequesters the I-*Sce*I:GR complex in the cytosol. Addition of dexamethasone allows the dissolution of the complex (Aoyama and Chua, 1997), liberating the I-*Sce*I:GR protein which can move to the nuclease and produce a DSB at the TL. In plants, an inducible I-*Sce*I was used to enhance intra-chromosomal recombination (Wehrkamp-Richter et al., 2009) and to perform targeted mutagenesis (Yang et al., 2009).

Plant GT strategies are generally based on the positive selection for GT events which repair a defective selectable marker. A DSB is induced at the TL inducing HR between a defective TL selectable marker gene and the repair DNA. For example in maize, D'Halluin et al (2008) re-transformed TL lines with a repair DNA and a construct encoding I-Scel, either delivering the DNA via particle bombardment or Agrobacteria. The frequency of GT versus random insertion, measured by acquisition of resistance to the herbicide BASTA, was up to 30% via particle bombardment and 3.7% using Agrobacterium. Shukla et al (2009) have also reported efficient GT in maize using Zn-finger nucleases. Although these studies show that GT is now possible in a major crop plant there is still the need to optimise GT to minimize the effort required to produce and identify GT events before GT becomes a routine tool for GM production.

A major limiting factor is the need to deliver the repair DNA and I-Scel encoding sequence efficiently into a large number of cells, which in the case of maize transformation can involve the transformation of many thousands of immature embryos or calli to obtain a few GT events. An attractive alternative is to create a few transformation events where the repair DNA and I-*Sce*I encoding sequence are stably but randomly integrated into the genome. The repair DNA is then controllably excised from the genome, and acts as a template for GT at the TL. This system has the advantage that every cell contains the repair template and thus, from a single transformed individual, a potentially unlimited number of GT experiments can be performed. Such a GT strategy has been successfully implemented in Drosophila, with the repair DNA being excised from the genome using the FLP recombinase and then linearised using I-*Sce*I (Huang et al., 2008; Rong, 2002) and has recently been reported also in Arabidopsis (Fauser et al., 2012).

In particular, Fauser et al (2012) describe an *in planta* GT system based on three different constructs that were transformed independently by floral dipping. The target locus contains a truncated β-glucuronidase (GUS) gene (uidA) that can be restored via GT. DSB induction at the two I-*Sce*I recognition sites flanking a kanamycin-resistance gene would result in excision of the kanamycin-resistance gene and in activation of the target locus for HR. The donor locus contains a GT cassette that is also flanked by two I-*Sce*I recognition sites, resulting in the release of a linear GT vector after I-*Sce*I expression. Homology between the activated target site and the GT vector sequence is 942 bp on one end and 614 bp on the other. In addition, the donor construct had 599 bp of sequence homology upstream and downstream of the I-*Sce*I sites, so that after excision of the GT vector, the resulting DSB could be repaired either by nonhomologous end joining (NHEJ) or by single-strand annealing (SSA).

In the supplementary figure S1, Fauser et al (2012) describe the general crossing schema. Single-copy target and donor lines were crossed, leading to a collection of different target/donor combinations. F3 plants, homozygous for both constructs, were crossed with the I-*Sce*I expression line Ubi::I-*Sce*I#10, leading to F1' plants that show somatic GT events (blue sectors after histochemical staining). Progeny of these plants (F2') was screened for germinal GT events (completely stained blue plants). Batches of seeds from different transgenic lines (F2') were grown on agar plates and then stained 14 d postgermination, making it possible to detect the roots of some individual seedlings which become bluish.

Consequently, the method of Fauser et al (2012) relies on the transfer of the GT events to the germ line; it comprises of three transformation steps, and multiple crossing steps (either between lines or self-crossing steps). An important point to note is that, the method described in Fauser et al (2012) requires self-crossing of the first generation plants obtained which contain the target site, the DSB-inducing enzyme and the donor gene.

In view of the above, routine use of GT in plants requires the development of efficient and inexpensive protocol. Low targeting efficiencies and the resulting need to produce and screen large numbers of transformed plants are major bottlenecks in the development and application of this technology.

The invention as described in the invention makes it possible to avoid the step of self-crossing the plants described in Fauser et al (2012), and which contain all elements that would lead to homologous recombination.

The invention makes use of the fact that
(i) the probability that one HR event would occur in at least one cell in a tissue when all elements needed for said event are present is high (if the tissue contains 10⁶ cells, there would be around 100 such cells)
(ii) plant cells are fully totipotent and it is thus possible to regenerate a plant from a single cell.

The invention proposes to solve the main problem which is to identify a cell where an HR event has occurred. For this, the invention proposes to (i) increase the number of such cells, and (ii) to increase the proportion of such cells in the studied sample.

Indeed, as described below, the method herein described comprises a step of cellular multiplication of cells, which will inevitably increase the number of cells where a HR event has occurred.

Furthermore, it is postulated that multiplication of cells by mitosis shall also induce occurrence of HR events in the progeny of a cell in which a HR event had not initially occurred, thereby increasing the proportion of cells in which a HR event has occurred in the multiplied cells.

Last, and in some embodiments, one can also use a selection marker, which will also increase said proportion.

In a specific embodiment, is described a GT system which avoids the necessity to achieve multiple plant transformation and crossing. In this system a dexamethasone inducible I-*Sce*I:GR protein excises a stably integrated transgene at the Donor Locus (DL), and cleaves the Target locus (TL), stimulating recombination repair of the TL using the excised transgene as template. GT results in the repair of a defective *npt*II gene conferring kanamycin resistance. The I-*Sce*I:GR protein successfully induces the appearance of kanamycin-resistant leaf tissue sectors. However no fully kanamycin resistant plants were detected in the progeny of these plants. Applying this GT strategy to tissue culture of embryo-derived TL/DL callus solved this problem and molecular analysis of 7 independent events showed that GT had occurred by ectopic recombination. With this approach a few stable transformation events can be used to generate a potentially unlimited number of GT events. The results furthermore show that cleavage of the DL is both unnecessary for targeted recombination, and a source of unwanted events when endonuclease cleavage is limiting. *In vivo* rare ectopic recombination events can thus be palliated by *in vitro* cells multiplication. This approach thus permits the isolation of GT plants in a practicable manner in plants, and specifically in economically important crop plants such as cereals, oily plants (rape, sunflower) or vegetables.

In a first aspect, the invention relates to a method to obtain a plant in the cells of which a homologous recombination event has occurred comprising the steps of
a) *in vitro* cellular multiplication of a plant tissue wherein said tissue comprises at least one cell able to express, when homologous recombination has occurred, an homologous recombination event marker
b) Selecting among multiplied cells, at least one cell expressing said homologous recombination event marker,
c) Regenerating a plant from the at least one cell obtained from step b) thereby obtaining a plant cell comprising an homologous recombination event in the genome of all of its cells.

Said selection step shall thus include the recovery of said selected at least one cell, in order to be able to regenerate a plant from this at least one cell.

It is to be noted that the plant is regenerated from the at least one cell that has been identified in step b). This (these) cell(s) express(es) the homologous recombination marker, thereby indicating that an homologous recombination event has occurred. Consequently, the plant thus regenerated is such that a recombination event is present in each and every of its cells. In a preferred embodiment, all cells contain the same recombination event, and the same genome (ie these cells all originate from the same clone).

This at least one cell, from which the plant is regenerated is any cell from the tissue that has been multiplied *in vitro.* This tissue may be or may not be (and preferably is not) a germinal tissue. Consequently, in some preferred embodiments, the recombination events that are present in the genome of the cells of the plant thus regenerated are not the result of the transfer of the recombination event to germinal cells as described in Fauser (*op. cit*.).

In a preferred embodiment, said homologous recombination event consists in targeted integration of a gene of interest in the genome of said cell by homologous recombination. In this embodiment, said gene of interest shall be integrated in the genome of said cell at a specific site that has been predetermined.

By "homologous recombination event marker", one means a marker which would allow detection of a cell (whether upon a specific stimulus or not) after the homologous recombination event has occurred.

This marker may be a gene that would be functional after the HR event has occurred (either by being fully inserted in the genome, or by being repaired / made functional / reconstituted upon HR), or a gene that has been deleted (in totality or partly) or made non-functional upon homologous recombination.

As an illustration of constructs that would produce a homologous recombination event marker, one can cite a gene, wherein two parts of the gene are present in the cell if no homologous recombination event has occurred and said gene is reconstituted upon homologous recombination. In this embodiment, each of the two parts of said gene presents a zone that is homologous (identical) to a sequence of the other part, thereby allowing homologous recombination to occur between these two zones. Another example is a gene which is effectively expressed when inserted by homologous recombination downstream of an endogenous promoter, thus allowing said gene expression driven by said endogenous promoter.

Another illustration of a homologous recombination marker lies in a suicide gene (ie a gene that will lead to death of the cell in presence of a particular stimulus), which is present and functional in the cell if any homologous recombination event has occurred. When such an event occurs, the gene is deleted or made non-functional, thereby making the cell resistant to the stimulus. One can cite the gene coding for thymidine kinase, which makes the cells sensible to ganciclovir, or cytosine deaminase which converts the 5-Fluorocytosine (5-FC, non-toxic) to the toxic 5-Fluoro-Uracile (5-FU). Other hybrid suicide genes such as Fcy::fur (combining the activities of cytosine deaminase (CD) and of uracile phosphoribosyltransferase (UPRT), sensibilizing cells to 5-FC), and Dck::umk (fusion between desoxycytidine kinase (DCK) and uridylate monophosphate kinase (UMK) metabolizing gemcitabine into toxic metabolites) have been developed by InvivoGen Therapeutics.

In some embodiment, said homologous recombination event marker consists of a marker gene that reacts and allows detection of the cell in which it is expressed upon application of a stimulus. Said stimulus may consist of adding a specific compound in the culture medium, said compound being metabolized by cells in which homologous recombination has occurred and which contain the functional marker gene and not in other cells (such compound may be a herbicide, an antibiotic or a compound which could be metabolized and color the cell). Said stimulus may consists of applying a specific wavelength to the cells, which would in turn emit another wavelength (this would be the case if the marker gene is such as the GFP (Green Fluorescent Protein) or Ds-Red).

Consequently, these selectable markers include, but are not limited to, antibiotic resistance genes, herbicide resistance genes or visible marker genes.

Broadly, any phenotypic marker that is known in the art may be used as the "homologous recombination marker" in this invention. A number of selective agents and resistance genes are known in the art. (See, for example, Hauptmann et al., 1988 ; Dekeyser et al., 1988 ; Eichholtz et al., 1987 ; and Meijer et al., 1991). Notably the marker used can be the *bar* or *pat* genes conferring resistance to bialaphos (White et al., 1990), the sulfonamide herbicide Asulam resistance gene, sul (described in WO 98/49316) encoding a type I dihydropterate synthase (DHPS), the *npt*II gene conferring resistance to a group of antibiotics including kanamycin, G418, paromomycin and neomycin (Bevan et al., 1983), the *hph* gene conferring resistance to hygromycin (Gritz et al., 1983), the EPSPS gene conferring tolerance to glyphosate (US 5,188,642), the HPPD gene conferring resistance to isoxazoles (WO 96/38567), the gene encoding for the GUS enzyme, the green fluorescent protein (GFP), expression of which, confers a recognizable physical characteristic to transformed cells, the chloramphenicol transferase gene, expression of which, detoxifies chloramphenicol.

It is clear that the recombination event shall lead to expression of said homologous recombination marker. Consequently, all elements allowing said expression shall be present, such as a promoter and a terminator sequence.

Homologous recombination is facilitated by double strand break (DSB) endonucleases. Endonucleases of the invention (also named restriction enzymes) can be for example an endonuclease cutting the genome at a specific site, like for example the meganuclease I-*Sce*I or as described in US20090271881. Specific endonuclease activity can also be obtained with the combination of a nuclease and a DNA-binding domain designed to recognize a specific target site in the genome in order to modify the DNA at the target site. Examples of such systems are the zinc-finger nuclease as described in Townsend et al, 2009, Nature 459:442-445 or Tal effector-nuclease as described in WO2011/072246.

In a preferred embodiment, said plant tissue is a tissue that has been isolated from a plant, a plantlet or a seed that has been obtained by crossing
(i) a donor plant, wherein said donor plant comprises, in its genome, a cassette comprising a gene of interest, wherein said gene of interest is flanked by two sequences X and Y that are identical or homologous to sequences X' and Y' present in the target plant,
(ii) and a target plant, wherein said target plant comprises, in its genome,
   a. sequences X' and Y', that are identical or homologous to said sequences X and Y, and
   b. a site R that can be recognized and cut by an endonuclease located between sequences X' and Y', wherein said site R is a rare site in the plant genome.

As intended herein and throughout this application, two sequences are said to be "homologous" if they present at least 90%, more preferably 95% even more preferably 100% identity. Preferably these sequences are at least 50, more preferably at least 100 nucleotides (nt) even more preferably at least 200 or at least 500 nt, even more preferably at least 1000 nt, or at least 2000 nt.

The person skilled in the art is able to determine the size of the fragment flanked by X' and Y' and which contains the endonuclease site. The literature describes various embodiments. As a matter of illustration, one can cite Fauser *et al* (2012, *op. ci*t.) where the fragment flanked by the X' and Y' sequences contains the *npt*II gene and is probably about 2-2.2 kb if only one I-*Sce*I sites is cut, whereas the distance between the sequences X' and Y' is much smaller (a couple hundred bases) in the examples of the present application.

As intended herein, a "rare" site is a site that has a low (< 0.1 %, more preferably <0.05 or 0.01 %) probability to be present in the genome of the plant. Preferably, it is present at most 10 times, preferably at most 5 times in the genome of the target plant.

Preferably, said site R is at least 15 bases, and preferably at least 18 bases long. Indeed, it is statistically demonstrated that the longer the sequence, the less chance it is present in a genome. In this case, the restriction enzyme may be recognized by a meganuclease, which is by definition a sequence-specific endonuclease with large (>12 bp) recognition sites.

In the context of the present invention, said meganuclease is preferably I-Scel, as used in Fauser (2012). Nevertheless, other meganucleases may also be used, such as HO, or the meganuclease described in Epinat et al (Nucleic Acids Research, 2003, Vol. 31 , No. 1 1 2952- 2962), in particular the hybrid meganuclease, in Chames et al (Nucleic Acids Res., 2005; 33(20): e178 - e178), or in Nomura et al (I-ApeI, Nucleic Acids Res., 2005; 33(13): e1 16 - e1 16.), or in Silva et al (I- DmoI, Nucleic Acids Res., 2004; 32(10): 3156 - 3168). One can also cite I-*Cre*I (Wang et al (1997) Nucleic Acids Res., 25, 3767-3776) or I-*Ceu*I (Marshall et al (1994) Eur. J. Biochem., 220, 855-859), which function as homodimers, or larger proteins bearing two (do)decapeptide motifs, such as I-Scel (Jacquier et al (1985) Cell, 41 , 383-394), PI--*Sce*I (Gimble, et al (1996) J. Mol. Biol., 263, 163-180) and I-*Dmo*I (Dalgaard et al. (1993) Proc. Natl Acad. Sci. USA, 90, 5414-5417).

I-*Sce*I.is a preferred meganuclease usable in the method of the invention.

The I-*Sce*I enzyme has been isolated from Saccharomyces cerevisiae and double strand breaks induced by this enzyme increase the rate of homologous recombination in mammalian cells (Choulika et ai, 1994, 1995 and WO9614408).

In a specific embodiment, said restriction site R is a DNA sequence which can be cut by a specific endonuclease which has been specifically developed to recognize and induce a double strand break at this site. Methods for generating endonuclease recognizing a specific DNA sequence have been developed, in particular by Cellectis (Romainville, France) and are described in particular in WO 2010/015899.

In view of the way the tissue that is cultured *in vitro* is obtained, it may contain, before multiplication, more than one cell that is able to express, when homologous recombination has occurred, the homologous recombination event marker. It may actually contain, before multiplication, one or a multiplicity of cells which already express said homologous recombination event marker. Consequently, multiplication will increase the number of such cells, as described above, which will make them easier to detect and isolate.

In a specific embodiment, said tissue is a somatic tissue, and is not a germinal tissue. In a preferred embodiment, said tissue is an immature embryo.

This embryo shall be excised from a seed that is obtained after crossing of the donor and target plants as described above.

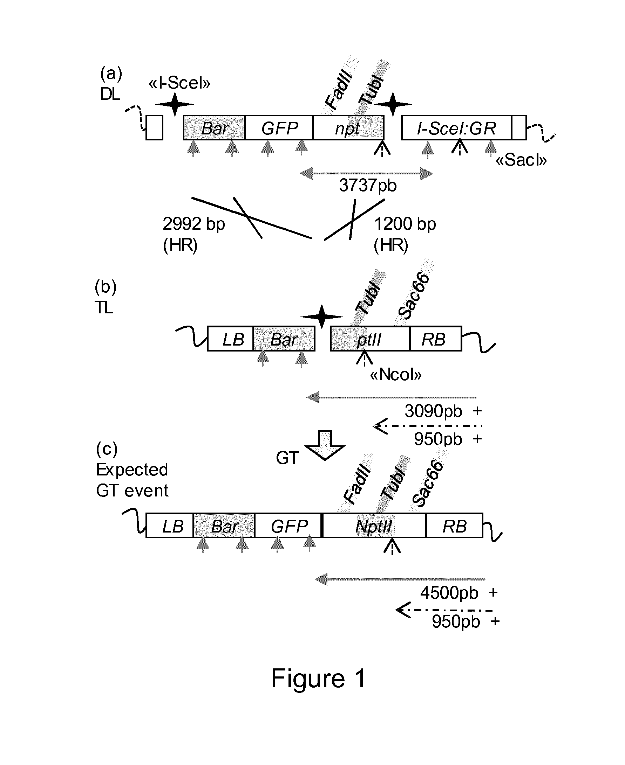

In a specific embodiment, the donor plant is such that it does not present, in its genome, any site R as present in the target plant. Indeed, as demonstrated below, it has been shown that it is not necessary to release, from the donor plant genome, the gene to be inserted in the target plant genome. This is surprising, as the prior art (see Figure 1 of Fauser et al, 2012) generally flanks said gene to be inserted with two R sites. Consequently, in methods of the prior art, the endonuclease shall both induce a double strand break at the target locus and release the gene to insert at the target site.

In a specific embodiment of the method according to the invention, it is preferred not to release said gene to insert. Consequently, the site R shall not be present in the donor plant genome. At the very least, should the R site be present in this genome, it should not flank the gene to insert.

In another embodiment, said tissue has been obtained after transformation of a target plant with a vector comprising a gene of interest, wherein said gene of interest is flanked by two sequences X and Y that are identical / homologous to sequences X' and Y' whereas sequences X' and Y' are present in the genome of said target plant, and whereas an endonuclease site is located between said sequences X' and Y',

In this embodiment, said vector may also comprise an expression cassette so that it allows expression of an endonuclease able to induce a double strand break at said endonuclease site of said target line. Alternatively, and as seen below the target plant may already comprise the expression cassette coding for the endonuclease in its genome, before transformation with the vector comprising a gene of interest flanked by sequences X and Y.

It is to be noted that, in this embodiment, the tissue that is multiplied *in vitro* is isolated from the T0 plants that are obtained after transformation of the target plant.

Methods for transforming a plant are known to one skilled in the art : methods of direct transfer of genes such as direct micro-injection into plant embryoids (Neuhaus et al., 1997), vacuum infiltration (Bechtold et al. 1993) or electroporation (Chupeau et al., 1989) or the bombardment by gun of particles covered with the plasmidic DNA of interest (Fromm et al., 1990, Finer et al., 1992). *Agrobacterium* mediated transformation methods may also be used *Agrobacterium tumefaciens,* in particular according to the method described in the article by An *et al*., (1986), or *Agrobacterium rhizogenes,* in particular according to the method described in the article by Guerche *et al*., (1987). According to a preferred mode, it is possible to use the method described by Ishida *et al.,* (1996) for the transformation of maize, or the method described in WO 00/63398 for the transformation of wheat.

In order for a homologous recombination event to occur, leading to targeted integration of a DNA sequence at a predetermined target site, the following event must take place:
- said predetermined target site must be provided
- said DNA sequence must be provided
- the flanking region of said DNA sequence must be homologous to sequences of the target site
- a double strand break must be performed at or near the target site

As a reminder a double strand break (DSB), is a lesion on both strands of a double strand DNA.

In the method of the invention, the double strand break is preferably induced by a cut made by the endonuclease. It is thus necessary to provide the said endonuclease.

Consequently, in a specific embodiment, the donor line further comprises, in its genome, an expression cassette which codes for said endonuclease. Consequently, said endonuclease shall be expressed and thus induce the double strand break at the site R of said target line.

In another embodiment, said expression cassette coding for said endonuclease is present in the target plant. In this embodiment, said target plant may have been obtained by crossing a first plant which comprises, in its genome, said sequences X' and Y' and said site R located between sequences X' and Y', and a second plant which comprises, in its genome, said expression cassette allowing expression of said endonuclease able to cut at said site R.

In this embodiment, it is preferred when said endonuclease is inducible.

The endonuclease may indeed be inducible. By "inducible", it is meant that the enzyme only becomes active in response to an external stimulus. This stimulus may be a chemical or mechanical stimulus.

In a preferred embodiment, the sequence encoding said endonuclease is under the control of an inducible promoter. As an illustration, the inducible promoter may be induced by a stress or a chemical agent.

Inducible promoters may be induced by pathogens or wounding, more preferably they are induced by an abiotic stress such as cold, heat, UV light, high salt and water deficit. Promoters useful for targeted expression in trangenesis are reviewed in Potenza et al., 2004. Some abiotic stress promoters are the *Arabidopsis thaliana* or *Oriza sativa* DREB genes promoters (Dubouzet et al., 2003; Lee et al., 2004 ; Pellegrineschi et al., 2004) ; the *Oriza sativa* SISAP1, CDPK7 or WSI gene promoters (Mukhopadhyay et ai, 2004 ; Saijo et ai, 2000; Takahashi et al., 1994) the *A. thaliana* rd29 gene promoters (Yamaguchi-Shinozaki and Shinozaki 1993). Some plant heat inducible promoters may also be used *hsp*18.2 or *hsp*101 from *A. thaliana* (Yoshida et al., 1995 ; Young et al., 2005 ), *hsp*17.6 or *hsp*17.3, from Glycine max (Severin and Schoffl, 1990 ; Saidi et al., 2005). DNA microarrays have been used to identify stress regulated sequences (Rabbani et ai, 2003 ; EP 1 452 596; WO 02/16655) The signalization pathway of the response to stress includes abscisic acid signalization so ABA-inducible promoters may also be powerful stress-inducible promoters, such as the *Hordeum* vulgare A22 and hvAI promoters (Shen et al., 1993 ; Straub et ai., 1994), *Zea maize* rab 17, DBF1 and DBF2 (Villardel et ai., 1990 ; Kizis and Pages, 2002), Arabidopsis thaliana ABF3 (Genbank accession AK175851), and *Oriza sativa* rab21 (Mundy and Chua, 1988).

In another embodiment, the foreseen promoters are induced by chemicals (for review, see Moore et al., 2006, Padidam M. 2003 and Wang et al., 2003 and Zuo and Chua 2000). Some examples of couples of chemically inducible systems and chemical inducer used in plants are, the alcA promoter from *A. nidulans*, inducible by the Ethanol (Roslan et ai., 2001) or the ecdysone receptor from *C*. *fumiferana*, inducible by an ecdysone agonist (Koo et al., 2004).

In another embodiment, the endonuclease consists of a fusion protein comprising a nucleic acid coding for said enzyme capable promoting the double strand break at site X, fused with a nucleic acid coding for Glucocorticoid Receptor (GR) Ligand Binding Domain (LBD) such as the rat GRLDB described in Miesfeld et al. 1986 and in WO 2007/135022. As indicated in WO 2007/135022, the modification to the endonuclease implies that it is not active without an external stimulus (application of dexamethasone), or less active than the non-modified restriction enzyme.

Upon application of this hormone, the restriction enzyme enters the nucleus of the cells, and performs the double strand break at its site of recognition.

In the method according to the invention, it is foreseen that cellular multiplication can be made by protoplast or callus culture. Methods for culturing plant cells are well known in the art.

In a specific environment, a selection compound is added to the culture medium at some point during tissue culture. This would allow selection of the cells in which the homologous recombination event has occurred, by selective multiplication of the cells in which said homologous recombination event marker is functional.

In another embodiment, the selection step includes the step of selection at least one cell expressing a reporter gene as described above (such as GFP or *lacZ).*

Regeneration of a plant from the cells that have been selected in vitro may be performed by any method known in the art.

Plant regeneration is the ability to regenerate an entire plant from either stem cells via organogenesis or via a single differentiated somatic cell (Birnbaum and Sánchez Alvarado (2008). Although this can occur in nature, for example via regeneration of plantlets from leaf edges of Kalanchoe via somatic embryogenesis (Garcês et al 2007), in crop plants this requires in-vitro culture. The principle is to place plant explants on nutritive media containing specific concentrations of hormones. The hormones such as cytokinins and auxins promote dedifferentiation, division and redifferentiation. Different ratios of hormones are required at different steps of regeneration.

The cells are placed in a medium containing growth factors and regulators such as benzyl adenine (BA) and indole acetic acid (IAA) in order to induce callus formation and the plants are then regenerated from said calli.

In the method according to the invention, it is preferred when:
a) if the tissue to multiply *in vitro* is issued from a cross between a target line and a donor line (F1), then said tissue is preferably an immature embryo that is isolated from a F1 seed
b) if the tissue to multiply *in vitro* is issued from a target line that has been transformed with a donor cassette, then said tissue is preferably a callus.

In a specific embodiment, said plant is a cereal, in particular maize or wheat.

Said regenerated plant may be self-crossed one or more times, in order to obtain a homozygous plant.

The invention also relates to a specific kit that is useful and especially designed for performing the method according to the invention. This kit for performing gene targeting (introduction of a gene of interest at a predetermined location in a target plant) in a plant, comprises
a) A donor plant, comprising in its genome, a cassette comprising a gene of interest, wherein said gene of interest is flanked by two sequences X and Y that are identical / homologous to sequences present at the predetermined location in the target plant,
b) A target plant, comprising, at said predetermined location in its genome, sequences X' and Y', that are identical / homologous to said sequences X and Y, and a site R located between sequences X' and Y', wherein said site R can be cut by an endonuclease,
wherein said cassette of the donor line does not comprise any of such site R.

As indicated above, said site R is rarely present in the genome of said target plant and is at least 15 bases, and preferably at least 18 bases long.

The donor and target plant are preferably cereal and in particular maize or wheat.

In a preferred embodiment according to the invention, said target plant is homozygous. In another embodiment, said target plant is not homozygous, but is homozygous for the target locus.

In a preferred embodiment, said donor plant is homozygous. In another embodiment, said donor plant is not homozygous, but is homozygous for the locus of the cassette comprising the gene of interest.

### DESCRIPTION OF THE FIGURES

Figure 1: Maps of the transgenic loci (not to scale): Scheme of the DL (a), of the TL (b) and of the expected GT event (c) with the position of the I-*Sce*I restriction sites (black stars). For Southern analysis, the sizes of DNA fragment hybridized with intOsTubI (Tubl), intfadII (Fadll) and TerSac66 (Sac66) were indicated for *Sac*I (full gray arrows) or by Ncol (black arrows)are also indicated on the maps( + indicate the size of supplementary genomic fragment).

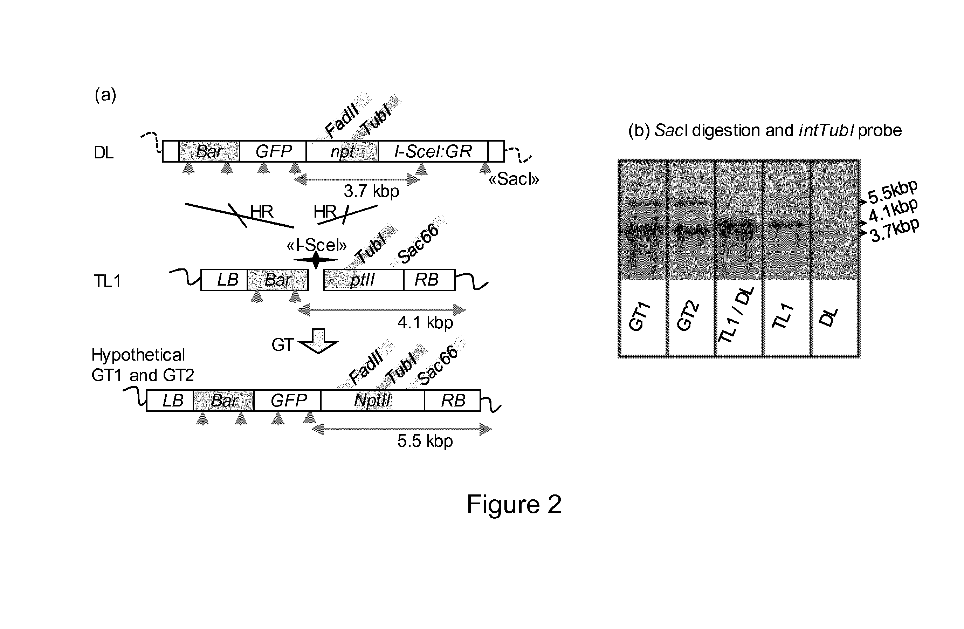

Figure 2: Southern blot results of GT1 and GT2 events: (a) Scheme of the hypothetical GT event occurring in the GT1 and GT2 events. Black stars indicate the excised I-Scel restriction site. The gray arrows indicate the *Sac*I restriction site positions and the size of DNA fragment containing the used probes. (b) Southern Blot analysis of control parental plants (TL1, DL, TL1/DL plants) and of recombinant GT plants from TL1/DL tissue culture (GT1 and GT2 lines) with *Sac*I-digested DNA and an intTubI specific probe. A fragment around 3,7kbp, indicative of the original DL, was detected in the in the GT1 and GT2 events. A 4,1kbp fragment, indicative of TL1 was not observed in the GT1 and GT2 events. The expected fragment of 5,5kbp, indicative of Nptll reconstitution due to HR between the DL and TL1, was detected in the GT1 and GT2 events. The DL intensive band indicated the homozygous state of the DL and the non-excision of the DL.

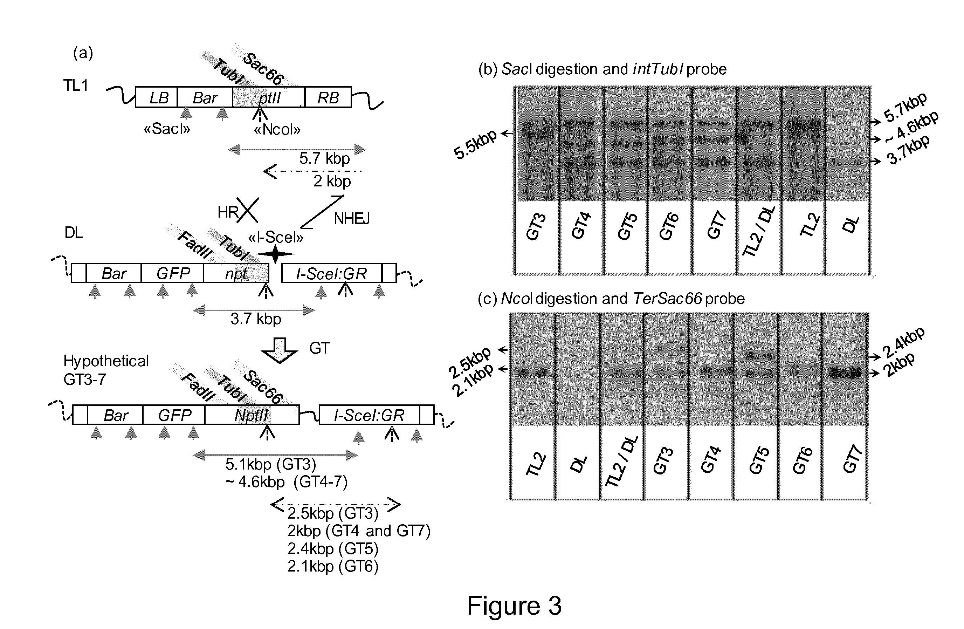

Figure 3: Southern blot results of GT3, GT4, GT5, GT6 and GT7 events: (a) Scheme of hypothetical GT events for GT3-7 events. Black stars represent the excised I-*Sce*I restriction site. The gray and black arrows indicate respectively the SacI and Ncol restriction site positions and the size of DNA fragment containing the used probes.. (b) Southern blot analyses of control parental plants (TL2, DL, TL2/DL) and of kanamycin resistant events derived from the TL2/DL (GT3-7) with Sacl-digested DNA and an intTubl specific probe. A 3,7kbp band, indicative of the non-excised DL, was detected in the GT4-7 events, but not in the GT3 event. The 5,7kbp fragment indicative of native TL2, was observed in the GT3-7 events. All putative GT events show an additional band around 5,5kbp for GT3 and around 4,6kbp for GT4-7 events (but not at the expected size of 7,1 kbp). (c) Southern Blot analysis of the same events with Ncol-digested DNA blotted with a terSac66 specific probe. The signal at 2kbp was observed for the TL2 and DL/TL2 controls. For the GT3, GT5 and GT6 events unexpected bands appeared at respectively 2,5kbp, 2,4kbp and 2,1 kbp.

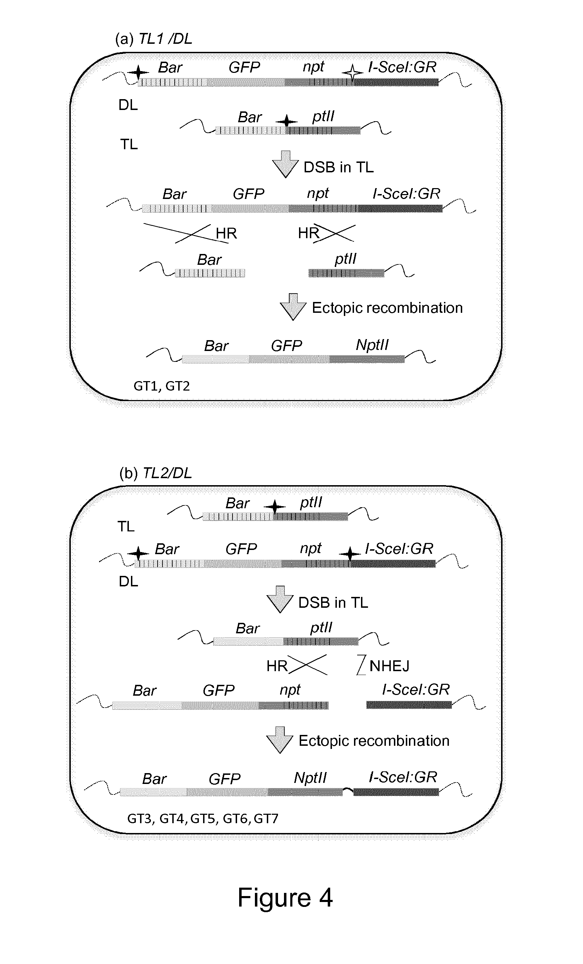

Figure 4: Scheme of the occurred events: The I-*Sce*I restriction sites were positioned on the map with black stars and the homologous regions with black hatching. The white star represents a mutated I-*Sce*I restriction site by NHEJ. (a) For the TL1/DL derived embryo the DL was integrated in the DSB occurred in the TL by ectopic recombination using the both homologous regions Bar and nptll. (b) For the TL2/DL derived embryo, the TL was integrated in DSB occurred in the defective nptll of the DL by ectopic recombination using the homologous region of nptll in one side and NHEJ in the other side.

### Examples

### Experimental procedures

### Production of GT constructs and Maize transgenic lines.

The binary vectors for the creation of the Target Locus, pBIOS-TL and Donor Locus, pBIOS-DL were constructed in the following manner. First in order to extend the region of homology between the truncated nptII genes in the TL and DL lines an 886bp rice Tubulin intron (GenBank, AJ488063) was introduced into the coding sequence of the NptII gene at position 204bp downstream of the ATG. A 5' truncated nptII-OsTubintron fragment lacking the first 150bp of the nptll coding region was cloned between an I-*Sce*I site and in front of the Arabidopsis Sac66 polyadenylation sequence (GenBank, AJ002532). The I-*Sce*I-3' nptll:OsTubint-AtSac66term fragment was then cloned into an SB11-based plant binary vector (Komari et al., 1996) containing a rice Actin promoter (McElroy et al., 1991) linked to the Bar selectable marker gene (White et al., 1990) and a Nos terminator, forming pBIOS-TL. To produce pBIOS-DL a 3' truncated nptII:OsTubintron fragment lacking the last 227bp of the nptll coding region was cloned behind the constitutive pSC4 promoter (Schünmann et al., 2003). A pSB11-based binary vector was created that contained the pOsActin-BAR-Nos term gene cassette and a CsVMV promoter (Verdaguer et al., 1998) linked to GFP, with both gene cassettes flanked by I-*Sce*I restriction sites. The pSc4-5' nptII:OsTublntron fragment was then cloned between the terminator of the GFP gene and the 3' I-*Sce*I site to complete the nptll repair region. Next the NLS-I-*Sce*I:GR gene (Wehrkamp-Richter et al., 2009), codon-optimised for maize expression, was cloned between a CsVMV promoter and 35SCaMV terminator. This cassette was then cloned between the nptll repair region 3' I-*Sce*I site and the RB to form pBIOS-DL. Agrobacterium tumefaciens strain LBA 4404 (pSB1) ((Hoekema et al., 1983) were transformed with pBIOS-TL and pBIOS-DL. For each construction, a clone containing the recombinant plasmid was selected. Embryos of maize inbred A188 were transformed with each construction and transformed plants were regenerated according to Ishida et al. (Ishida et al., 1996) using glufosinate selection.

### Plant analysis

Genomic DNA was extracted from leaves by using the DNeasy 96 plant kit (Qiagen). Genomic DNA (10µg) was digested, separated on 1% agarose gel by electrophoresis, transferred to nylon membrane and hybridised to 32P marked probes following strand procedures ((Sambrook and Russell, 2006)). The FST of transgenes were amplified using an adapter-anchor PCR method according to the method of (Balzergue et al., 2001) modified by (Sallaud et al., 2003)) using DNA digested with Sspl or Pvull. Plant genotyping was performed by PCR using a DNeasy 96 plant kit (Qiagen). To amplify fragments longer than 2kb a TakaraLaTaq kit (Takara) was used. GFP fluorescence of sampled plant leaves was visualized under a fluorescence stereomicroscope (Leica MZ16F) using a GFP2 filter.

### Crossing, culture and treatment of transformed plants

Plants were grown in a greenhouse with a 16h day at 26°c, 400µE m-2s-1 and an 8 hours night at 18°c. Dexamethasone treatment on plants were realised by immersion of the seed during 2 days in a solution of 30µM dexamethasone. Kanamycin treatments were performed by the application of 50µl of a solution at 200mg.I-1 kanamycin and 1% tween on the apical region of 2 weeks old plants.

### Somatic embryogenesis

Embryos isolated from selfed plants containing the TL and DL were placed onto a regeneration medium according to Ishida et al. (Ishida et al., 1996) lacking the kanamycin selective agent. For first experiment, LS-AS medium was complemented by 0, 30 or 50µM dexamethasone and after one week transferred sequentially to LSD1.5, LSZ and 1/2LSF medium lacking dexamethasone and containing 50 mg.L-1 of kanamycin. For the second experiment, callus was developed for 3 days on LS-AS, 1 week on LSD1.5 and 3 weeks on LSZ medium. Then, callus was cultivated 1 week on LSZ medium complemented by 0 µM, 30 µM or 50µM of dexamethasone and selected for 2 weeks on LSZ and then ½ LSF medium complemented by 50mg.L-1 of kanamycin. GFP fluorescence of callus was visualized under the fluorescence stereomicroscope (Leica MZ16F) with a GFP2 filter.

### Callus analysis

PCR was performed directly on callus tissues using Terra direct PCR polymerase (Ozyme) in 20µl with specific TL and DL primers. Genomic DNA was extracted from pooled callus of same genotype and dexamethasone treatment by using an DNeasy 96 plant kit (Qiagen). Primers were designed according to GS FLX Titanium emPCR LIBL kit (Roche) with a specific TAG for each condition. PCR were performed using Platinum Taq DNA Polymerase High Fidelity (Invitrogen). Emulsion PCR was realised on the obtained PCR produces with emPCR Emulsion kit (Roche). PCR produces were sequenced with emPCR sequencing kit (Roche) with Genome Sequencer FLX (Roche). Sequences from each condition were independently assembled and aligned to the reference sequence containing the non-mutated I-*Sce*I site. Sequences differences of 1 or 2 bp with the reference sequence found in the TL genotype, lacking I-*Sce*I-GR were discarded since these are likely to be sequencing errors. Sequence differences of 3 or more base pairs encompassing the I-*Sce*I site were identified and manually regrouped per condition to identify the number of independent mutations per condition.

### Results:

### The GT test system:

Two plant transformation constructs, the TL (Target Locus) construct and the DL_I-*Sce*I:GR (Donor Locus) construct were developed to test the GT strategy (figure 1). The T-DNA of the TL construct contains the plant transformation selectable marker gene Bar followed by an I-*Sce*I restriction site and the 3' part of the npII gene (figure 1b). The T-DNA of the DL construct contains a dexamethasone-activatable maize codon optimized I-Scel gene (I-*Sce*I:GR) and an nptll repair region bordered by two I-*Sce*I restriction sites (figure 1a). The nptII repair region contains the Bar gene, the GFP gene and a 5' part of the nptll gene. The GFP gene here serves as a mock gene of interest and additionally allows easy identification of DL-containing plants. The nptll repair region and the TL share common sequences of 2992bp in the Bar region and 1200bp in the nptll region, provided largely by the insertion of an intron (intTubI) into the defective nptII genes. This homology should allow homologous recombination between these two sequences and the consequent repair of the Nptll gene, resulting in kanamycin resistance (figure 1c). The TL and the DL constructs were independently transformed into maize to generate TL and DL lines respectively. Two intact TL (TL1 and TL2) lines and one DL line, each containing a single copy of the transgene were selected by Southern Blotting analysis (not shown) and their genomic flanking sequences isolated (see supplementary sequence data). The DL line expressed both GFP and the I-*Sce*I:GR transcript. The two TL lines were then selfed in order to isolate homozygous descendants which were then crossed with the DL line. The F1 progenies and their descendents were selfed. The segregation of the TL and the DL indicate that two constructions were not genetically linked.

### Detection of Somatic repair of NptII in TL/DL leaves.

For each TL line crossed with the DL line, the F1 progeny containing the TL and the DL were identified by PCR analysis and separated in two groups, one treated with dexamethasone to induce I-*Sce*I activity and other not. Plants were grown and DNA extracted from leaves. To detect excision of the repair DNA from the DL, PCR was performed using primers positioned on either side of the DL I-Scel restriction sites. A total of 12 untreated and 7 dexamethasone treated plants were analysed and 3 excision events were detected for each condition, indicating a basal activity and non-significant inducibility of I-*Sce*I:GR in these conditions.

The analysed F1 plants were then selfed to identify kanamycin resistant plants among the F2 descendants. To detect the presence of the TL and the DL, 176 F2 (42 for the TL1/DL line and 134 for the TL2/DL line) plants were analysed by PCR: 21 TL1/DL and 55 TL2/DL descendants contained the both TL and DL. Kanamycin was applied to the apical meristematic region of wild type (WT) and F2 plants. On WT plants and F2 descendants containing only the TL or the DL, this resulted in bleaching of the developing leaf. However, leaves with green sectors within the kanamycin bleached zones were observed in 60% of plants carrying both the TL and DL, corresponding to 38% of TL1/DL and 70% of TL2/DL plants. PCR analysis performed on the DNA extracted from the green sectors permitted amplification and sequencing of the repaired Nptll gene, but not from the DNA extracted from bleached or untreated leaf sectors. Other batches of F2 seeds were sown, and none of the additional 504 descendants were fully kanamycin resistant, however green resistant sectors were again observed in TL/DL plants.

### Recovery of fully kanamycin-resistant plants via in vitro tissue culture

No fully kanamycin-resistant progeny were identified in 680 F2 plantlets containing the both TL and DL, so to circumvent this problem a tissue culture approach was used. Plant regeneration from maize leaves has not been reported but calli derived from immature maize embryos are routinely used to regenerate plants (Lu et al., 1983). Embryos isolated from immature kernels of selfed F2 plants containing the TL and the DL were placed on callus induction medium with dexamethasone at 0µM (control), 30µM or 50µM. From 2356 extracted embryos (619 from the TL1/DL and 1737 from the TL2/DL plants), 7 independent kanamycin resistant GT events (Table 1) were recovered and shown to carry a repaired nptll gene which was amplified by PCR and sequenced. Two were obtained from the TL1/DL embryos and five from the TL2/DL embryos (GT3, GT4, GT5, GT6 and GT7). GT efficiencies calculated as the number of GT events per immature embryo range from to 0.13% to 0.55% (Table 1).

**Table 1: Table summarizing gene targeting experiment**

| **Lines** | **Extracted embryos** | **Dexa-methasone concentration µM** | **Number of kanamycin resistant events** | **Name of kanamycin resistant events** | **GT Frequency, discovered kanamycin resistant events / extracted embryos** |
|---|---|---|---|---|---|
| *TL1* x *DL* | 183 | 0 | 0 | / | 0% |
| | 255 | 30 | 1 | GT1 | 0,39% |
| | 181 | 50 | 1 | GT2 | 0,55% |
| *TL2* x *DL* | 800 | 0 | 1 | GT4 | 0,13% |
| | 563 | 30 | 3 | GT3, GT5, GT6 | 0,53% |
| | 374 | 50 | 1 | G7 (+) | 0,27% |

Only 1 of the 7 GT events was obtained from non-treated control embryos and dexamethasone treatment thus appears to increase the number of GT events. The numbers of events are however low and as we observed somatic recombination during development of F2 plants (green sectors, above) in the absence of dexamethasone treatment, probably come from a basal leaky I-*Sce*I:GR activity. We thus tested the effect of dexamethosone treatment on TL DSB induction through measurement of mutations in the TL I-*Sce*I site. Embryos of F2 plants with green resistant sectors were extracted (14 from the TL1/DL and 69 from the TL2/DL) for somatic embryogenesis. A sample of the callus formed from each embryo was analysed by PCR to determine the presence of the DL and the TL. Each callus was divided in three parts, one part placed on medium without dexamethasone, one on medium with 30µM and one on medium with 50µM dexamethasone, for one week. Samples of TL/DL calli from each treatment and of the TL calli was pooled for DNA extraction (Table 2). A 400bp region around the I-*Sce*I TL restriction site was amplified from each pool and sequenced by 454 sequencing. Approximately 15000 sequences were obtained and analysed to estimate the mutation rate at the I-*Sce*I restriction site due to NHEJ repair. No mutations were detected in the absence of the DL (TL controls which do not carry the I-*Sce*I gene). In the TL/DL lines mutations of the TL I-*Sce*I site were detected, with the number of independent mutations increasing 3.5 to 5 fold with 30µM and 5 to 6 fold with 50µM of dexamethasone. These data thus confirm a basal acitivity of I-*Sce*I:GR in inducing mutations in the I-*Sce*I target site and that dexamethasone treatment increases I-*Sce*I:GR activity in calli (table 2). The presence of mutations in the target in the absence of dexamethosone however shows a certain leakiness in this system.

**Table 2: Number of mutations at the I-Scel site of TL, according to genotype and dexamethasone treatment**

| **Lines** | **Embryo** | **Genotype** | **Dexamethasone concentration µM** | **Mutations in TL I-*Sce*I site** |
|---|---|---|---|---|
| TL1 x DL | 14 | TL1 | / | 0 |
| | | TL1 / DL | 0 | 15 |
| | | | 30 | 76 |
| | | | 50 | 76 |
| TL2 x DL | 69 | TL2 | / | 0 |
| | | TL2 / DL | 0 | 6 |
| | | | 30 | 15 |
| | | | 50 | 36 |

### GT at the Target Locus: Analysis of the kanamycin resistant events obtained from TL1/DL plants

Two GT events were identified from calli from TL1/DL plants. Southern analysis was carried out on genomic DNA of regenerated GT1 and GT2 plants, digested with SacI and hybridized with three different probes: intFadII (present in the DL and predicted to be present in a GT locus), intTubl (common to the DL, TL and predicted to be in the GT locus) and terSac66 (present in the TL and predicted to be in the GT locus). The Southern blot results with the intTubl probe are shown in figure 2b. A band of 3.7kbp was detected in the DL lane and a 4.1kbp band in the TL1 lane, both bands were observed in the DLxTL1 control lane. As expected, in GT1 and GT2 lanes the TL1 band disappeared and a new 5.5kbp band, also observed with intFadII and terSac66 probes (supplementary data), was detected, confirming Nptll repair at the TL. The non-excised DL band was observed at 3.7kbp with an intensity consistent with a homozygous state. The GT1 and GT2 plants were backcrossed to wild-type plants twice and kanamycin resistance was inherited as a single Mendelian locus. In the first back-cross, 53% and 55% of GT1 and GT2 descendants were kanamycin resistant and all presented the non-excised DL, confirming that GT1 and GT2 are heterozygotes for the reconstructed (by GT) Nptll gene at the TL. For the second back-cross, 35% and 36% of GT1 and GT2 descendants were kanamycin resistant. All resistant plants expressed GFP and PCR amplification confirmed presence of the intFadII and terSac66 regions, and absence of the TL1-specific fragment containing the I-*Sce*I TL1 site (supplementary data) which together confirm the expected reconstitution of Nptll. Among the kanamycin resistant descendants, 61% of GT1 and 55% of GT2 also contained all sequences specific to the DL (LB I-Scel, RB I-Scel and I-*Sce*I:GR). Finally, PCR fragments were amplified from kanamycin resistant GT1 and GT2 plants containing only the GT locus and sequenced, Primers were designed in the terSac66 and in the genomic flanking sequence isolated from the TL1 LB, so as to amplify the complete reconstructed Nptll gene in the target locus. The sequence of the amplified fragment obtained (supplementary data) was identical to that predicted for HR between the DL and TL1 resulting in repair of Nptll at the TL1. Analysis of the GT1 and GT2 events thus showed that they are true GT events at the TL and that the GT was not associated with excision of the donor sequence from the DL in either case, suggesting that they arose through ectopic recombination (Puchta, 1999) between the TL and DL (figure 2a).

### GT at the Donor Locus: Analysis of the kanamycin resistant events obtained from TL2/DL plants

Analysis of the GT3, GT4, GT5, GT6 and GT7 events revealed a second class of GT events which result from reconstitution of *npt*II at the DL rather than at the TL. Again the mechanism was ectopic recombination between the TL and the DL but in this case involving a single cross-over to repair *npt*II at the DL followed by integration of a variable length of TL sequence via NHEJ or MHEJ (Micro Homology End Joining) into the DL (figure 3a). The evidence for this conclusion is as follows. The DNA of each event was digested with *Sac*I and hybridized with intFadII, intTubl and terSac66 probes. The results with the intTubl probe are presented in figure 3b. The original DL and TL2 bands of 3.7 kbp and 5.7 kbp respectively were detected in the GT samples, except for GT3 which lacked the original DL. However the predicted GT-specific band of 7.1kbp for GT at the TL was not detected in the GT lanes. Instead a band, also observed with intFadII and terSac66 probes (supplementary data), was observed at 5.5 kbp for GT3 and around 4.6 kbp for GT4, GT5, GT6 and GT7, indicating a different mechanism of nptll repair. These findings were confirmed by Southern blotting of *Nco*I digested DNA probed with the terSac66 probe (figure 3c). The TL band of 2 kbp was found unchanged in all GT lanes and an additional band was observed in GT3, GT5 and GT6 lanes. The presence of the terSac66 on two different DNA fragments indicates that either one copy of a potentially homozygous TL2 was modified, but not via the expected double crossover, or that the TL2 was used as template by HR to repair an I-*Sce*I:GR induced DSB in the DL (figure 3a). The fact that PCR of the GT3 line could not detect a DL lacking the *npt*II repair fragment (data not shown) and that GT3 lacks a band specific to the original DL supports the idea that in GT3, at least, the DL has been modified. To shed further light on the mechanism, the GT events were backcrossed twice to the wild-type and analyzed by PCR (supplementary data). Kanamycin resistance was inherited as a single locus. Resistance was not correlated to the presence of the LB TL amplicon which is specific to the TL and predicted to be present in a true GT event at the TL. Amplification of the LB TL and TL I-*Sce*I amplicons in 46% of the GT4, 44% of the GT5 plants and about 17% of the GT6 events can be attributed to the presence of a segregating unmodified TL in these plants. Resistance was strictly correlated with the presence of DL sequences either side of the I-*Sce*I restriction site next to the defective *npt*II in the DL. However a PCR fragment (DL I-*Sce*I), of the expected size, across this I-*Sce*I site could not be amplified. This suggests either deletion around this I-*Sce*I site or the insertion of a sequence including the TL terSac66 into this I-*Sce*I site. This latter hypothesis was confirmed by amplification and sequencing of the GT loci using primers located in the intFadII and in the I-*Sce*I:GR gene. Analysis of the amplified sequence (supplementary data) showed an HR event restoring the Nptll gene on one side and a NHEJ or MHEJ event copying and linking a part of the TL2 flanking sequence to the I-*Sce*I:GR promoter on the other side. For the GT3 event, after the region of homology in the *npt*II gene, 909bp of the TL2 corresponding to the missing part of the defective *npt*II (including the terSac66) and 502bp of its genomic flanking sequence of the TL2 RB were linked by NHEJ to the other side of the break which had lost 52bp of DL sequence. For GT5, the event is similar to the GT3 event but only 197bp of the TL2 flanking sequence was copied and 9bp of the break were deleted to repair this side by NHEJ. Regarding the GT4 event, a Holliday junction might have been formed on the other side from a micro-homology of 4bp, thus 882bp of the TL2 comprising the missing part of the Nptll (including the terSac66) was copied in the repair sequence with 55bp deleted from the DL. In conclusion the sizes of these sequences of these GT events correspond to the observed sizes of bands seen on the Southern Blots.

### Discussion

These examples describe the development of a tool for precise remobilization of a transgene randomly inserted into the maize genome by its excision, recombination and insertion into a defined genomic site. This strategy was tested by crossing of stably transformed TL and DL maize lines containing 3' and 5' overlapping regions of an nptll gene respectively. Induction of I-*Sce*I activity in these lines with dexamethosone was used both to create a DSB at the TL and also release the nptII repair DNA from the DL. HR of the liberated nptII repair DNA with the TL would then reconstitute the *npt*II gene and also mobilise a GFP gene into the TL. Kanamycin selection allows selection of putative GT events.

Testing of 680 F2 progeny of dexamethasone treated (or not) F1 plants carrying the TL and DL, did not permit identification of any kanamycin resistant plants, suggesting that germinal or early meristematic GT events are very rare under the conditions tested.

However, In the course of testing these plants for kanamycin resistance, the presence of green kanamycin-resistant sectors on the kanamycin-bleached leaves was noted, suggesting the presence of somatic HR events between the TL and DL. DNA extracted from these green sectors, but not bleached leaf regions, could be used to amplify a restored functional Nptll gene. Such green resistant sectors on bleached plants have previously been described in tobacco plants carrying an intra-chromosomal HR reporter based on Nptll reconstitution (Peterhans et al., 1990), and also in Arabidopsis (Christian et al., 2010). Other studies of GT based on Nptll restoration and selection of resistant plants through addition of kanamycin to the culture medium in tobacco (Puchta, 1999) and *Arabidopsis* (Vergunst et al., 1998), did not however report green resistant sectors.

In maize, it was shown here that application of kanamycin to the apex permits the detection and quantification of somatic GT events in leaves without affecting survival of the (sensitive) plants. Multiple kanamycin treatments are possible and progeny can be obtained from treated plants. This assay, which should be applicable to other plant species, is currently being used to test and optimise GT frequencies.

In tobacco lines containing the equivalent of our TL, retransformed with a repair sequence and constitutive I-*Sce*I, the observed GT frequency increased proportionally with the expression level of I-*Sce*I (Puchta et al., 1996). Similarly, in our maize plants the frequency of green kanamycin resistant sectors gives direct information about I-*Sce*I:GR activity.

Given that *npt*II repair sequence excision from DL was observed in equivalent proportions from maize plants grown in the absence or the presence of dexamethasone treatment, there is clearly basal activity of I-*Sce*I:GR in the maize leaves and dexamethasone does not further induce I-*Sce*I:GR in the tested conditions.

In a previous study with I-SceI:GR in *Arabidopsis,* basal activity was found but expression could be induced around 25 to 200 fold when dexamethasone was supplied in the growth medium (Wehrkamp-Richter et al., 2009). It is speculated that the dexamethasone applied to maize germinating seed does not penetrate into the seed in sufficient quantities to further induce I-*Sce*I:GR activity. Dexamethasone treatment does however induce I-*Sce*I:GR activity when added to the callus growth medium, where a 3.5 to 6 fold increase in the number of mutations at the TL correlated to the I-*Sce*I:GR activity (Table 2).

Notwithstanding the GT observed in somatic tissues, no kanamycin resistant plants were found in the 680 tested F2 progeny of the TL/DL lines. A strategy based on tissue culture selection and regeneration of kanamycin-resistant plants from TL/DL calli was thus tested. This approach permitted the selection of 7 independent GT events in two separate experiments involving a total of 2356 embryos (table 1). Two of these, GT1 and GT2, were generated from embryos from TL1/DL plants; molecular and genetic analyses confirmed that they are true GT in which the TL has been modified by ectopic recombination using the DL as template. The remaining five events (GT3, GT4, GT5, GT6 and GT7) were generated from TL2/DL line embryos; southern and sequence analyses showed that they result from modification of the DL, using the TL as template. The mechanism appears to be the creation of a DSB by I-*Sce*I:GR in the DL I-*Sce*I site next to the 5' *npt*II region. Recombination of the *Npt*II side of the break with the homologous TL2 as donor creates a functional *Npt*II at the DL. However the other side of the break does not carry homology to the TL2, and thus must be repaired by NHEJ or MMEJ (GT4) resulting in variable lengths of TL2 sequence integrated into the DL. Such HR+NHEJ gene conversion events have been previously reported in plants (Puchta, 1999).

This surprising difference in the nature of the GT events identified in the calli from the two parent lines led us to sequence the TL and DL of these lines. This analysis identified a mutation which eliminates the right side I-*Sce*I cut site of the DL of the TL1/DL line (between nptll and I-*Sce*I:GR - see Figure 4a). In the TL1/DL calli therefore, in contrast to the TL2/DL calli, I-*Sce*I:GR can only cleave the DL. Although the numbers of GT events analysed are low, this difference very probably explains the different types of GT events identified in calli from the two lines. In the TL1/DL calli, recombination initiated by I-*Sce*I cleavage of the DL would not generate a functional NptII gene and so only events initiated by cleavage in the TL would be selected. In the TL2/DL calli however, recombination initiation through cleavage adjacent to the Nptll sequences in either the TL or the DL could result in reconstruction of Nptll (Figure 4b). In the TL2/DL calli, identification of (only) recombination events in which the DL was recipient clearly shows that single, incomplete I-*Sce*I cleavage of the DL is frequent in these cells.

These data thus clearly show that only cleavage of the TL is needed for successful GT in these plants, as well as illustrating the risk of including multiple I-*Sce*I restriction sites in plants in which I-*Sce*I expression/activity is limiting. The basal level of I-*Sce*I cleavage in the absence of dexamethasone induction further compounds this risk through elevated levels of mutation in the I-*Sce*I sites of the DL. The dependence of this problem on limited I-*Sce*I activity would thus explain the difference with recent studies in Arabidopsis using a comparable strategy, in which only clean GT events were found (Fauser et al., 2012). They observed efficient repair DNA excision, probably due to efficient expression of the optimized I-*Sce*I gene and GT was observed in up to 1% of the progeny. Limited endonuclease activity is however a common problem in experiments of this type (Puchta et al., 1996). In Drosophila, Gong et al (2003) also reported low I-*Sce*I mediated repair fragment excision and estimated that excision occurred in 7% of cells. They thus used the FLP recombinase to excise a circular repair DNA from the genome that was subsequently linearised by I-*Sce*I. This system gave good GT rates in Drosophila but has not thus far been shown to work in maize (Yang et al., 2009).

A GT system based on I-*Sce*I mediated cleavage of the target, and excision of the *npt*II repair region from the genome has been tested in maize, the rationale being that an excised repair DNA should more easily find its target. The presence of both the target and the donor in the plant means that this approach avoids the need for (and dependence on) retransformation with the donor sequence and I-Scel (D'Halluin et al., 2008) or Zinc finger nuclease (Shukla et al., 2009) encoding sequences. A strategy in which multiplication of the cells carrying the TL was developed, the DL and I-*Sce*I encoding sequences permits selection of rare ectopic recombination events. With this approach a few stable transformation events can be used to generate a potentially unlimited number of GT events, of particular interest in cases where plant transformation frequencies are a limiting factor. These results furthermore show that cleavage of the donor locus is both unnecessary for targeted recombination, and a source of unwanted events when endonuclease cleavage is limiting.

## Claims

1. A method to obtain a plant in which an homologous recombination event has occurred comprising the steps of
a) *in vitro* cellular multiplication of a plant tissue wherein said tissue comprises at least one cell able to express, when homologous recombination has occurred, an homologous recombination event marker
b) Selecting among multiplied cells, at least one cell expressing said homologous recombination event marker,
c) Regenerating a plant from the at least one cell obtained from step b) thereby obtaining a plant comprising an homologous recombination event in the genome of all of its cells.

2. The method of claim 1, wherein said homologous recombination event consists in targeted integration of a gene of interest in the genome of said cell by homologous recombination.

3. The method of claim 1, wherein said tissue comprises a multiplicity of cells expressing said homologous recombination event marker.

4. The method of any of claims 1 to 3, wherein said homologous recombination event markers is chosen among a gene that is reconstituted or rendered functional after said homologous recombination event and a gene that is deleted or rendered non-fonctional / after said homologous recombination event.

5. The method of any of claims 1 to 4, wherein said tissue has been isolated from a plant that has been obtained by crossing a donor plant, wherein said donor plant comprises, in its genome, a cassette comprising a gene of interest, wherein said gene of interest is flanked by two sequences X and Y that are identical / homologous to sequences present in the target line, and a target plant, wherein said target plant comprises, in its genome, sequences X' and Y', that are identical / homologous to said sequences X and Y, and a endonuclease site located between sequences X' and Y'.

6. The method of claim 5, wherein said donor plant does not present, in its genome, any endonuclease site as present in said target line.

7. The method of any of claim 5 or 6, wherein the donor line further comprises, in its genome, an expression cassette allowing expression of an endonuclease able to cut at said endonuclease site of said target line.

8. The method of any of claims 1 to 4, wherein said tissue has been obtained after transformation of a target plant, which comprises in its genome sequences X' and Y' and a endonuclease site located between sequences X' and Y', with a vector comprising a gene of interest, wherein said gene of interest is flanked by two sequences X and Y that are identical / homologous to said sequences X' and Y'.

9. The method of claim 8, wherein said vector also comprises an expression cassette allowing expression of an endonuclease able to induce a double strand break at said endonuclease site of said target line.

10. The method of any of claim 5 to 9, wherein said target plant has been obtained by crossing a first plant which comprises, in its genome, said sequences X' and Y', that are identical / homologous to said sequences X and Y, and a endonuclease site located between sequences X' and Y', and a second plant which comprises, in its genome, an expression cassette allowing expression of an endonuclease able to cut at said endonuclease site [upon stimulation /inducible promoter].

11. The method of any of claims 5 to 10, wherein said endonuclease is I-Scel.

12. The method of any of claims 1 to 12, wherein said tissue is an immature embryo.

13. The method of any of claims 1 to 12, wherein cellular multiplication is made by protoplast or callus culture.

14. The method of any of claims 1 to 13, wherein cellular multiplication is made on a selective media allowing the selective multiplication of the cells in which said homologous recombination event marker is functional.

15. The method of any of claims 1 to 13, wherein the selection step consists in selecting at least one cell expressing a reporter gene.

16. The method of any of claims 1 to 15, wherein said plant is a cereal.

17. The method of any of claims 1 to 16, wherein said plant is maize or wheat.

18. A kit for performing gene targeting [introduction of a gene of interest at a predetermined location in a target plant] in a plant, comprising
a) A donor plant, comprising in its genome, a cassette comprising a gene of interest, wherein said gene of interest is flanked by two sequences X and Y that are identical / homologous to sequences present at the predetermined location in the target plant,
b) A target plant, comprising, at said predetermined location in its genome, sequences X' and Y', that are identical / homologous to said sequences X and Y, and a endonuclease site located between sequences X' and Y',
wherein said cassette of the donor line does not comprise any [ou does comprise none] of said endonuclease site.

19. The kit of claim 18, wherein said donor and target plant are cereal.

20. The kit of claim 19, wherein said donor and target plant are maize.

21. The kit of claim 19, wherein said donor and target plant are wheat.
